(19) Europäisches Patentamt

European Patent Office

Office européen des brevets

(11) **EP 0 959 912 B1**

(12) **FASCICULE DE BREVET EUROPEEN**

(45) Date de publication et mention
de la délivrance du brevet:
**29.09.2004   Bulletin 2004/40**

(51) Int Cl.⁷: **A61M 1/10**, A61M 1/12

(21) Numéro de dépôt: **97906220.5**

(86) Numéro de dépôt international:
**PCT/FR1997/000303**

(22) Date de dépôt: **19.02.1997**

(87) Numéro de publication internationale:
**WO 1997/030740 (28.08.1997 Gazette 1997/37)**

(54) **GENERATEUR DE PRESSION POUR DISPOSITIF D'ASSISTANCE CARDIAQUE A CONTREPRESSION**

DRUCKGENERATOR FÜR EINE HERZUNTERSTÜTZUNGSVORRICHTUNG

PRESSURE GENERATOR FOR A COUNTERPRESSURE CARDIAC ASSISTANCE DEVICE

(84) Etats contractants désignés:
**AT BE CH DE DK ES FI FR GB GR IE IT LI LU MC NL PT SE**

(30) Priorité:  **21.02.1996  FR 9602136**

(43) Date de publication de la demande:
**01.12.1999   Bulletin 1999/48**

(73) Titulaire: **Synthelabo Biomedical, S.A.**
**92350 Le Plessis-Robinson (FR)**

(72) Inventeur: **FRANCHI, Pierre**
**F-94400 Vitry-sur-Seine (FR)**

(74) Mandataire: **Dupuis-Latour, Dominique**
**SEP Pagenberg & Associés**
**14 boulevard Malesherbes**
**75008 Paris (FR)**

(56) Documents cités:
**FR-A- 1 184 203          US-A- 4 938 766**

## Description

L'invention concerne un dispositif d'assistance cardiaque implantable du type à ballonnet de contrepression.

La technique du ballonnet intraaortique de contrepression est bien connue pour apporter une assistance hémodynamique efficace au ventricule gauche en cas d'insuffisance cardiaque congestive : le ballonnet, introduit dans la branche descendante de l'aorte, est gonflé pendant la phase diastolique du cycle cardiaque et injecte de ce fait un volume de sang supplémentaire dans le réseau artériel en amont et en aval de sa position. Dégonflé pendant la systole cardiaque consécutive, il diminue la charge du ventricule gauche et permet ainsi d'accroître le débit sanguin. Le bilan hémodynamique est positif : augmentation de la fraction d'éjection, diminution de la pression télédiastolique. Ainsi, le ballonnet délivre le complément d'énergie que le ventricule n'est plus en mesure de fournir, et l'état du patient est très sensiblement amélioré.

On a déjà proposé des systèmes implantés permettant de mettre en oeuvre cette technique de façon entièrement autonome, comme cela est par exemple décrit dans le US-A-5 222 980, ou encore dans le EP-A-0 876 168 (WO-A-97/26929), publié le 31 juillet 1997 et faisant donc partie de l'état de la technique seulement au titre de l'art. 54(3) CBE, et intitulé *Pompe d'assistance cardiaque implantable du type à ballonnet de contrepression*.

Ces documents décrivent tous deux une pompe d'assistance cardiaque implantable permanente insérée dans l'aorte descendante, fonctionnant sur le principe précité du ballonnet de contrepression et constituée d'une membrane souple et élastique ayant la forme d'un manchon dont l'axe est confondu avec celui de l'aorte et disposé en lieu et place d'un tronçon d'aorte réséqué. La membrane est contenue dans une chambre rigide épousant sensiblement la forme de la membrane au repos, et dans laquelle l'injection d'un fluide hydraulique en provenance d'un générateur externe a pour effet de comprimer la membrane et donc de réduire le volume de sang qu'elle contient. À l'inverse, l'extraction du fluide provoque l'augmentation de son volume intérieur, donc le remplissage de la pompe.

Plus précisément, la présente invention a pour objet, dans ce système, le générateur permettant de contrôler les variations de pression du fluide hydraulique, et donc le fonctionnement de la pompe.

L'un des buts de l'invention est de proposer une structure de générateur qui optimise le bilan énergétique global de la pompe afin, d'une part, de limiter la consommation énergétique du dispositif implanté et, d'autre part et surtout, de faire fonctionner la pompe de la manière la plus physiologique possible, en générant dans l'ensemble du système cardiovasculaire un débit sanguin présentant une caractéristique la plus proche possible de celle d'un organisme sain. On verra que le générateur de l'invention permet d'atteindre ces buts tout en étant, du point de vue mécanique, de structure simple, robuste et compacte, caractéristiques indispensables pour un système implanté.

On verra également, selon un autre aspect, que le dispositif de l'invention peut être utilisé de façon purement passive, c'est-à-dire sans apporter globalement d'énergie supplémentaire au système (il ne comporte pas de moyens moteurs, ou ces moyens sont inactifs), le coeur subvenant seul à la demande énergétique. On verra que dans ce cas le dispositif de l'invention permet néanmoins de modifier de façon avantageuse l'onde de pression en se comportant alors essentiellement comme une élastance (facteur d'élasticité) additionnelle venant par exemple compenser la dégradation de l'élasticité naturelle du réseau artériel.

Dans ce dernier cas, le dispositif de l'invention se comporte essentiellement en régulateur de pression, et c'est en ce sens que l'on utilisera la terminologie générique "dispositif générateur/régulateur" pour englober les deux mises en oeuvre possible de l'invention, respectivement active ou passive. On notera d'ailleurs que le même dispositif peut être utilisé, selon les périodes, soit en mode actif soit en mode passif, par exemple en le commutant en mode actif dans les phases d'activité et en mode passif pendant les phases de repos.

Pour atteindre les buts précités, l'invention propose un dispositif décrit par le US-A-5 222 980 précité, et comportant les éléments énoncés par la revendication 1. Les sous-revendications visent des formes de mise en oeuvre particulières avantageuses.

D'autres caractéristiques de l'invention apparaîtront à la lecture de la description ci-dessous d'un exemple de mise en oeuvre.

La figure 1 est une vue schématique montrant la pompe d'assistance cardiaque, ses éléments associés et l'environnement dans lequel est implanté cet ensemble.

La figure 2 est une vue schématique de la pompe d'assistance cardiaque et du dispositif de l'invention, permettant d'expliquer la manière dont ces deux éléments coopèrent.

La figure 3 illustre la caractéristique effort/déformation du ressort de compensation, considéré isolément, du dispositif de la figure 2.

La figure 4 est une caractéristique donnant la pression artérielle en fonction du déplacement du piston du dispositif de la figure 2, en l'absence de ressort de compensation.

La figure 5 est homologue des figure 3 et 4 combinées ensemble, c'est-à-dire lorsque l'on utilise le ressort de compensation dans le dispositif en situation.

La figure 6 est homologue de la figure 5, dans une autre configuration de mise en oeuvre.

La figure 7 est une vue plus détaillée montrant en coupe la structure interne du dispositif de l'invention.

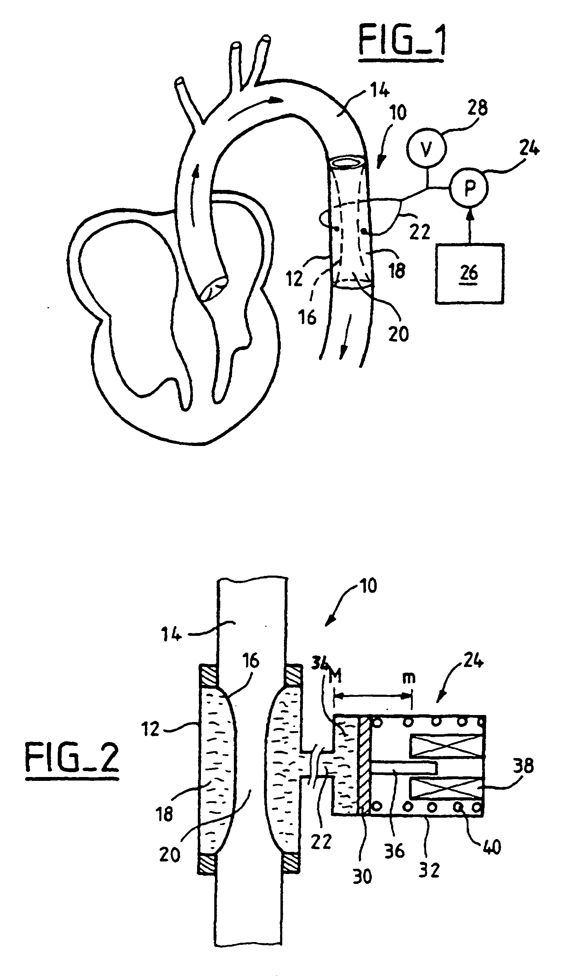

Sur la figure 1, on a représenté une pompe d'assistance cardiaque implantable, de type en elle-même connu (par exemple d'après le US-A-5 222 980 ou le WO-A-97/26929 précités), dont l'élément principal 10 comprend un corps rigide 12, typiquement en forme de cylindre de révolution, ouvert à ses deux extrémités et inséré dans l'aorte descendante 14, l'axe de l'aorte et l'axe du corps 12 étant confondus et ces deux éléments étant sensiblement de même diamètre.

Le corps rigide 12 contient une membrane souple 16. Dans la forme de réalisation illustrée, la membrane 16 a au repos une forme homologue de celle du corps 12, de manière à épouser sensiblement la forme de ce dernier, auquel elle est solidarisée aux deux extrémités sur toute sa périphérie.

On définit ainsi entre le corps 12 et la membrane 16 un espace intermédiaire 18, fermé, de volume variable et, à l'intérieur de la membrane 16, un espace central 20, également de volume variable, ce dernier volume diminuant lorsqu'augmente le volume 18, et inversement.

L'augmentation de volume de l'espace 18 résulte de l'injection d'un fluide hydraulique (typiquement une solution saline aqueuse biocompatible, par exemple un sérum physiologique) en un ou, de préférence, plusieurs points reliés par l'intermédiaire d'une conduite 22 à une source de pression variable 24 contrôlée par une électronique de commande 26. On peut avantageusement prévoir en outre un réservoir de flux hydraulique 28 sous forme d'un septum accessible par voie percutanée au moyen d'une aiguille hypodermique pour permettre l'ajustement du volume et/ou de la salinité du fluide, ou le vidanger.

L'invention a plus particulièrement pour objet l'élément 24, c'est-à-dire le dispositif générateur/régulateur de pression du système implanté. Le schéma de principe de ce dispositif 24 est illustré figure 2. Celui-ci comprend un piston 30 mobile dans un corps cylindrique 32 entre deux positions extrêmes distantes M et m de manière à définir un volume variable 34 en communication de fluide, via la conduite 22, avec l'espace 18 de la pompe, volume dont la variation vient donc agir sur la membrane 16.

La position M correspond à la course maximale du piston, donc au minimum du volume 34, au maximum du volume 18 et à la compression maximale de la membrane 16 ; cette position extrême correspond à la fin de la diastole cardiaque, où la pression aortique atteint la valeur la plus basse du cycle cardiaque. L'autre position extrême, m, correspond au maximum du volume 34 et donc au minimum du volume 18 ; elle est atteinte en cours de systole, où la pression aortique atteint la valeur la plus haute du cycle cardiaque.

Le piston 30 est actionné par l'intermédiaire de son axe 36 qui est lui-même entraîné par un moteur 38, typiquement un moteur électrique piloté par l'électronique 26 en fonction de divers signaux recueillis. Le piston reçoit également l'effort communiqué par un ressort 40 venant appuyer sur sa face intérieure et le sollicitant vers la position de déplacement maximal M.

La pression exercée par le fluide hydraulique sur le piston 30 est égale à la pression aortique, l'influence de l'inertie du sang et du fluide, des pertes par viscosité de ces fluides et de la résistance élastique de la membrane pouvant être négligée parce qu'elle est minimisée du fait de l'architecture de la pompe.

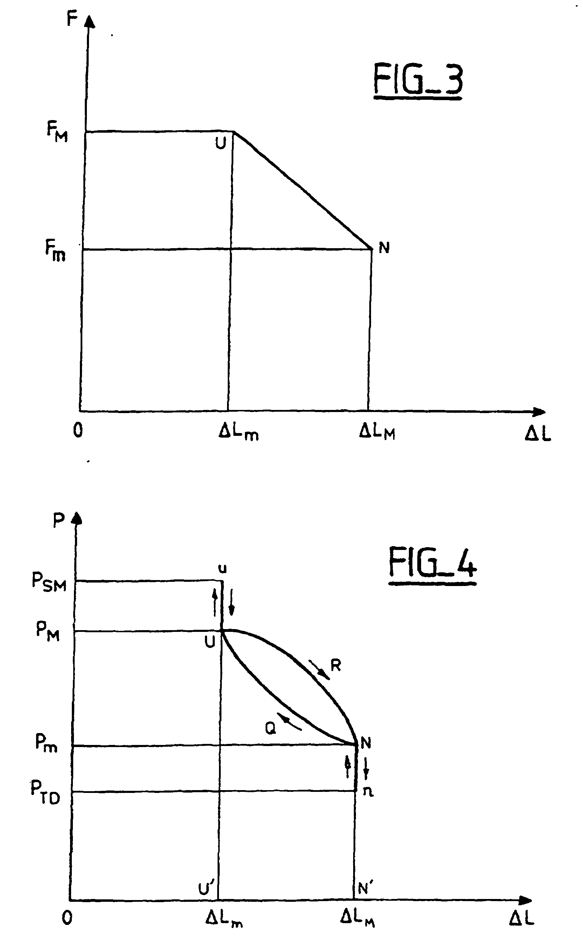

On va maintenant expliquer le fonctionnement du dispositif, en référence aux figure 3 à 6.

Pour mémoire et pour la clarté de l'exposé, la figure 3 illustre la caractéristique effort/allongement (F/$\Delta$L) du ressort 40 (supposée linéaire) considérée isolément entre les deux valeurs extrêmes $\Delta L_m$ et $\Delta L_M$ correspondant respectivement aux positions m et M portées sur la figure 2; les efforts exercés correspondants sont $F_m$ et $F_M$.

Les figures 4 à 6, quant à elles, donnent la pression $P = F/\Sigma$ du fluide exercée sur la face frontale (de surface $\Sigma$) du piston 30 et donc sensiblement égale à la pression aortique, en fonction du déplacement $\Delta L$ du piston entre les deux valeurs extrêmes $\Delta L_m$ et $\Delta L_M$ précitées.

On va tout d'abord étudier le fonctionnement du dispositif en faisant abstraction du ressort 40 (figure 4) : en fin de diastole et en début de systole, le point de fonctionnement du piston se situe en n. Pendant la phase systolique, la pression artérielle passe de la valeur $P_{TD}$, pression télédiastolique la plus basse du cycle, à la valeur $P_{SM}$, pression systolique maximale, en suivant le chemin nNQUu. Entre les points N et U le piston se déplace d'une extrémité à l'autre, de $\Delta L_M$ à $\Delta L_m$, tandis que de n à N et de U à u il ne se déplace pas.

L'énergie fournie par le milieu sanguin au dispositif est représentée par l'aire de la boucle N'nNQUuUU'N', qui est égale à celle de la boucle N'NQUU'N'. La diastole produit un parcours inversé ramenant le point de fonctionnement à la position initiale n par le chemin uURNn. De façon similaire à la précédente, l'énergie fournie par le dispositif au milieu sanguin est représentée par l'aire de la boucle U'URNN'U'.

Le bilan énergétique d'un cycle se traduit, dans le cas de la figure 4, par une énergie fournie par le dispositif au milieu sanguin égale à l'aire de la boucle NQURN (si cette boucle était parcourue dans le sens anti-horaire, c'est le milieu sanguin qui fournirait au dispositif l'énergie correspondante).

La difficulté pour le dispositif, du point de vue de son propre bilan énergétique, est que les pertes soient minimales, problème d'autant plus difficile à résoudre que les énergies échangées sont sensiblement plus élevées que l'énergie différentielle transférée. En effet, un dispositif électromécanique dont le moteur est électrique, par exemple un électroaimant, ou un moteur tournant associé à un convertisseur de mouvement rotatif en mouvement linéaire, développera un travail suivant le chemin URN avec un rendement assez bon, par exemple 0,8, et une perte proportionnelle à l'aire U'URNN' et recueillera de l'énergie dans le sens inverse suivant le chemin NQU avec un rendement sensiblement plus mauvais, par exemple 0,5, et une perte proportionnelle à l'aire N'NQUU'.

En conséquence le bilan énergétique est très mauvais. En appelant $W_Q$ le travail recueilli et $W_R$ le travail fourni, l'énergie recueillie est :

$$e_Q = \rho_1 \, W_Q \qquad \rho_1 = 0,5$$

et l'énergie dépensée est :

$$e_R = W_R/\rho_2 \qquad \rho_2 = 0,8$$

Si $W_R = 1,2 \, W_Q$, cas pratique dans le cadre de l'application envisagée, le travail différentiel obtenu est $0,2 \, W_Q$ pour le bilan énergétique suivant :

$$e_R - e_Q = (1,2 \, W_Q/0,8) - 0,5 \, W_Q = W_Q$$

tandis que le travail fourni est $0,2 \, W_Q$. Le rendement de l'opération est $(0,2 \, W_Q)/W_Q = 0,2$.

Si les pressions $P_{SM}$ et $P_{TD}$ étaient telles que $P_{SM} < P_M$ et/ou $P_{TD} > P_m$, la boucle de travail différentiel résultant serait bornée par u et/ou n au lieu de U et N, mais le raisonnement serait semblable et le résultat identique.

Le dispositif objet de l'invention propose la solution suivante.

Prenant en considération le mouvement alternatif du piston au cours du cycle, passant du point de fonctionnement N au point U, l'effort communiqué au piston peut être obtenu par deux efforts agissant en série dans l'axe du piston :

1°) Un élément passif élastique, par exemple le ressort hélicoïdal 40 tel que représenté figure 2, qui emmagasine et restitue la majeure partie de l'énergie échangée. Lorsque le ressort est prévu dans le système, la caractéristique globale de ce dernier est modifiée et devient une combinaison des caractéristiques des figures 3 (ressort seul) et 4 (système sans ressort).

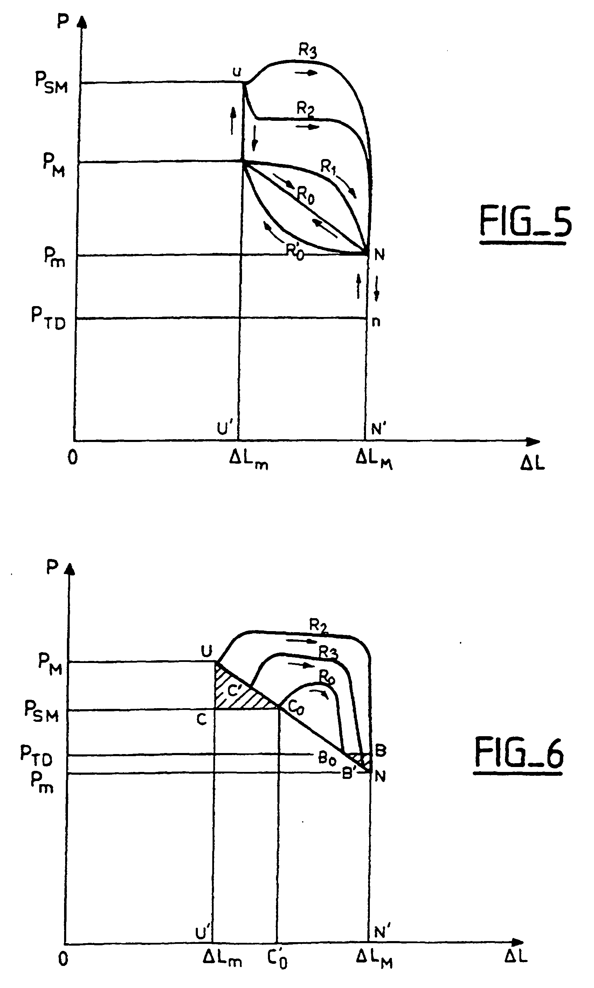

Cette caractéristique globale est illustrée figure 5 : le point de fonctionnement du ressort évolue entre N et U suivant le chemin $R_o$ dans le sens $NR_oU$ à la systole et $UR_oN$ à la diastole. Ce chemin est rectiligne si le ressort est linéaire, il peut prendre une forme légèrement différente s'il n'est pas linéaire, sans conséquence sur le fonctionnement. L'avantage du ressort est que son bilan énergétique est voisin de 1.

2°) Un élément électromécanique apportant les efforts nécessaires pour modifier le chemin bouclé entre N et U. Par exemple sur la figure 5 le chemin $nNR_oUu$, inchangé, pour la systole, et le chemin $uUR_1Nn$ pour la diastole, forment une boucle $NR_oR_1N$ qui représente le travail transmis directement par l'élément électromécanique au piston, donc au milieu sanguin, indépendamment de l'énergie échangée correspondant à l'aire de la boucle $U'UR_oNN'U'$, cet échange étant supporté par le seul ressort.

En conséquence les énergies brassées par le moteur sont sensiblement inférieures à ce qu'elles étaient dans le cas de la figure 4, les pertes sont donc réduites, les dimensions du moteur le sont aussi, le problème de récupération d'énergie à mauvais rendement ne se pose plus. Pour la bonne compréhension du mécanisme de passage du chemin $UR_oN$ au chemin $UR_1N$, on notera que le second chemin est parcouru en un temps plus court que le premier, l'augmentation de la pression se traduisant par une augmentation de la vitesse de déplacement du piston et par suite une augmentation du débit diastolique instantané de l'aorte.

En conclusion, sur les bases de l'exemple explicité plus haut, le travail transmis étant $0,2 \, W_Q$, et l'énergie consommée étant :

$$e_R = (0,2 \, W_Q)/0,8 = 0,25 \, W_Q$$

le rendement global devient :

$$\rho_G = (0,2 \, W_Q)/(0,25 \, W_Q) = 0,8$$

valeur à comparer avec le rendement de 0,2 du cas précédent.

En variante du cas considéré ci-dessus, la figure 5 présente d'autres chemins correspondant à des énergies croissantes délivrées par le dispositif au milieu sanguin : chemins $UR_2N$, $uR_3N$ résultant d'un effort ajouté à celui du ressort représenté par la différence de pression entre le chemin $uR_3N$, par exemple, et le chemin $UR_oN$ qui est celui du ressort. On peut aussi concevoir que le moteur électrique corrige le processus systolique, empruntant le chemin $NR'_oU$ au lieu de $NR_oU$, le moteur électrique apportant un travail correspondant à l'aire de la boucle $NR'_oUR_oN$, qui se soustrait du travail du ressort, le bilan se traduisant par une énergie moindre empruntée au milieu sanguin correspondant à l'aire $N'NR'_oUU'$. Ce chemin n'est évidemment pas souhaitable du point de vue du bilan énergétique du dispositif, mais il pourrait l'être pour d'autres raisons. La forme de la boucle sera en définitive déterminée par un compromis entre différentes conditions d'ordre physiologique, énergétique, mécanique et électrique.

En autre variante, la figure 6 représente le cas où $P_{SM} < P_M$ et $P_{TD} > P_m$. En ce cas le parcours libre du ressort non assisté par le moteur est $B_oC_o$, limitant donc la course du piston. La contribution du moteur peut s'exprimer par le chemin diastolique $C_oR_oB_o$, sans modification de la course du piston.

Cependant le moteur peut ramener la course du piston à l'extrémité correspondant à $\Delta L_m$, déplaçant la position du piston de $C_o$ en U en fin de systole. Pour effectuer ce déplacement le moteur exerce une force croissante de 0 en $C_o$ à $P_M$-$P_{SM}$ en U, fournissant un travail représenté par l'aire $C_oCUC_o$, le milieu sanguin fournissant le travail correspondant à l'aire $C'_oC_oCUC'_o$.

De façon identique, le point $B_o$ peut être ramené sur l'extrémité N, moyennant un travail fourni par le moteur électrique et représenté par l'aire $B_oBN$. Ainsi, moyennant une énergie supplémentaire représentée par la somme des deux aires des triangles $C_oCU$ et $B_o$-BN, le dispositif peut transférer au milieu sanguin l'énergie correspondant à l'aire $NUR_2N$. L'opération qui consiste à augmenter le transfert d'énergie au milieu sanguin de la valeur correspondant à l'aire $B_oC_oR_oB_o$ à la valeur correspondant à l'aire $NUR_2N$, peut s'effectuer dans de bonnes conditions de rendement énergétique malgré le défaut d'adaptation du ressort à la dynamique de pression artérielle représentée par $P_{TD}/P_{SM}$, le ressort étant trop raide.

On notera enfin que, de la même manière, les positions du piston auraient pu être amenées sur des points intermédiaires C' entre $C_o$ et U et B' entre $B_o$ et N, donnant lieu à un chemin diastolique $C'R_3B'$.

On remarquera enfin qu'il existe un cas où seule l'élasticité du ressort serait nécessaire au dispositif, sans transfert d'énergie au milieu sanguin. C'est celui où l'on souhaite réduire la post-charge du ventricule gauche en augmentant l'élasticité apparente de l'aorte par accroissement de l'éjection systolique du ventricule gauche grâce au volume emmagasiné par le piston pendant la systole. Le dispositif est alors simplifié par suppression du moteur électrique. Il devient passif et peut, par exemple, équiper une prothèse aortique qui, grâce à la grande élasticité apparente que lui confère le dispositif, peut présenter l'avantage important de diminuer la post-charge du coeur, et par conséquent la tension systolique.

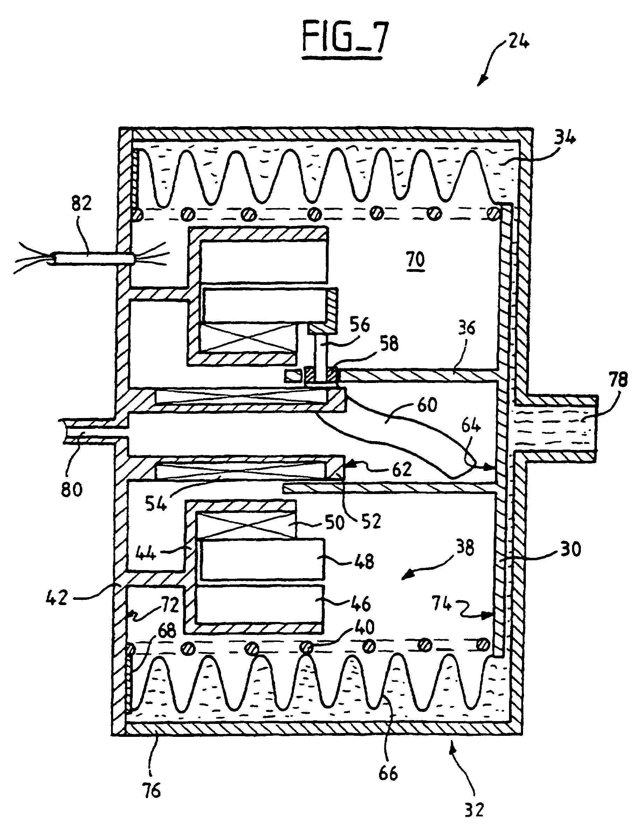

La figure 7 illustre, en coupe, un exemple de structure du dispositif générateur/régulateur de pression 24.

Dans cet exemple de réalisation, le corps 32 a la forme d'un boîtier sensiblement cylindrique dont la face postérieure est un flasque 42 pourvu, côté intérieur, d'un support 44 pour le moteur électrique 38, qui comprend essentiellement un stator 46 et un rotor 48 montés sur les roulements à billes circulaires 50. Le moteur est avantageusement un moteur électrique du type moteur-couple, sans balai, commandé par commutation électrique des bobinages du stator. La rotation de ce type de moteur (et donc le fonctionnement du dispositif) peut être contrôlée de façon très précise, et il peut être en outre pourvu de capteurs de déplacement par exemple du type à effet Hall ou optiques, qui permettent de connaître très précisément la position angulaire relative du rotor par rapport au stator et donc, comme on l'expliquera par la suite, la position du piston 30.

En position axiale, le flasque 42 porte un tube de guidage cylindrique 52 sur lequel coulisse l'axe 36 du piston, de préférence en forme de tube creux, le coulissement de ce tube 36 sur le guide 52 étant facilité par des roulements à billes linéaires 54.

Le rotor 48 est solidaire d'un doigt radial 56 tourné vers l'intérieur, qui porte à son extrémité un galet 58 s'engageant dans une gorge hélicoïdale 60 gravée dans l'arbre 36.

De cette manière, le mouvement du rotor provoque un déplacement axial de l'arbre 36, et donc du piston 30, par l'intermédiaire du galet 58 et de la gorge hélicoïdale 60.

Les dimensions des différents éléments, notamment le pas de la gorge hélicoïdale 60, sont choisis pour que la transmission soit réversible, c'est-à-dire que non seulement le déplacement du rotor entraîne une translation de l'axe 36 du piston, mais, en sens inverse, une sollicitation du piston (par la pression exercée sur la face en contact avec le fluide hydraulique) n'est pas entravée par l'ensemble gorge-galet.

Le déplacement du piston 30 vers l'arrière, c'est-à-dire en direction du flasque 42, est limitée par la venue en butée de la face frontale 62 du guide 52 avec la face interne 64 du piston 30.

Le piston proprement dit 30 fait partie d'un ensemble comportant notamment un soufflet 66 soudé en périphérie du piston à son extrémité frontale et terminé, à son autre extrémité, par un disque 68 de fixation contre le flasque 42, de manière étanche. L'ensemble formé par le piston 30, le soufflet 66 et le flasque 42 définit une chambre 70 isolée de l'environnement extérieur, notamment du volume 34 situé entre cet ensemble piston-soufflet et le reste du corps 32 et contenant le fluide hydraulique. Le ressort hélicoïdal 40 est disposé dans ce volume fermé 70, et il appuie à l'une de ses extrémités, en 72, sur la paroi intérieure du flasque 42 et à l'autre extrémité, en 74, sur la face interne du piston 30.

Ce ressort 40 assure la majeure partie de la composante élastique du dispositif dont l'intérêt a été décrit plus haut en relation aux figures 5 et 6, le soufflet 66, qui peut être un soufflet métallique, venant éventuellement jouer un rôle élastique supplémentaire.

On peut éventuellement prévoir des moyens (non représentés) pour ajuster la compression du ressort hélicoïdal 40 par décalage de son extrémité postérieure par rapport à la face interne 72 du flasque 42 sur laquelle il est appuyé. Ce décalage peut être réalisé par l'intermédiaire d'un disque appuyé sur l'extrémité du ressort et solidaire d'un manchon rigide disposé à l'intérieur du ressort hélicoïdal et au voisinage immédiat de celui-ci, déplacé axialement à l'aide d'un micromoteur électrique, de manière à ajuster et optimiser la plage de fonctionnement de l'élément élastique en fonction de la tension artérielle.

Les parois latérales et frontales du corps 32 sont constituées par un couvercle 76 venant s'appliquer contre le flasque 42 de manière à définir le volume 34 rempli par le fluide hydraulique, ce couvercle étant muni d'un abouchement 78 relié à la pompe, comme cela a été illustré sur la figure 2 précitée.

Le volume intérieur 70 de l'ensemble piston-soufflet est rempli d'air ou de gaz en atmosphère contrôlée et peut être relié par une buse 80 à une chambre de pression de référence, dont la pression sera maintenue très proche de la pression atmosphérique.

Enfin, un câble électrique multiconducteur 82 assure l'alimentation électrique du moteur et la transmission des données, notamment les données de mesure de déplacement du piston.

Dans une variante simplifiée du dispositif que l'on vient de décrire, on peut omettre le moteur électrique 38, ainsi que son support 44, le tube 52 et l'arbre creux 36. Les pièces subsistantes sont essentiellement le flasque 42 équipé de la buse 80, le boîtier 76 équipé de l'abouchement 78, le soufflet 66 et le ressort hélicoïdal 40.

Le dispositif est en ce cas passif. L'ensemble 10 a la même structure que dans le cas précédent mais, fonctionnellement, n'est plus véritablement une "pompe", mais simplement une prothèse aortique, et le dispositif de l'invention joue le rôle d'élastance artificielle branchée sur cette prothèse aortique, le coeur subvenant seul à la demande énergétique.

Dans cette variante, le dispositif simplifié permet de compenser le durcissement des artères, l'élasticité accrue (de manière artificielle) du système artériel évitant de demander une puissance excessive au ventricule pour vaincre la charge opposée par l'inertie et la résistance de la colonne sanguine qu'il doit mettre en mouvement, et permettant ainsi de procurer à même énergie, une hémodynamique améliorée.

On notera incidemment que ces remarques s'appliquent également au cas d'un dispositif complet, c'est-à-dire motorisé, mais non alimenté, par exemple lors des phases de repos, pendant lesquelles un complément énergétique n'est pas nécessaire au coeur pour subvenir aux besoins de l'organisme. On voit que, même dans cette configuration où le moteur est arrêté, le dispositif apporte par sa seule présence un bénéfice en procurant artificiellement un surcroît d'élasticité à l'aorte.

**Revendications**

1. Un dispositif intraaortique d'assistance cardiaque implantable du type à ballonnet de contrepression, du type comprenant :

   - une pompe (10) comprenant un corps (12) apte à être inséré dans une artère, notamment l'artère aorte descendante, définissant un premier volume (20) apte à être traversé par le sang à cet endroit et pourvu d'une membrane souple (16) définissant entre corps et membrane un second volume (18) séparé du premier volume par la membrane, ce second volume étant un volume variable permettant de modifier cycliquement et de manière contrôlée le premier volume (20), ce dernier diminuant lorsqu'augmente le second volume, et inversement ; et
   - un générateur/régulateur de pression (24) comportant une enceinte déformable définissant un troisième volume (34) en communication de fluide avec le second volume (18),

   dispositif **caractérisé en ce que** le générateur/régulateur de pression (24) comporte en outre des moyens formant ressort (40) aptes à solliciter l'enceinte déformable à l'encontre d'un accroissement du troisième volume résultant d'une augmentation de la pression dans le second volume (18) et, corrélativement, dans l'enceinte,
   de manière à procurer un surcroît d'élastance à l'artère au cours du cycle cardiaque.

2. Le dispositif de la revendication 1, dans lequel l'enceinte déformable comprend un piston (30) mobile dans un corps (32), les moyens formant ressort (40) sollicitent le piston dans le corps dans le sens de la diminution du troisième volume, et le piston est mobile entre deux positions, une position (m) de volume maximal et de compression maximale du ressort et une position (M) de volume minimal et de compression minimale du ressort.

3. Le dispositif de la revendication 1, comprenant en outre des moyens moteurs (38) pour solliciter de manière contrôlée l'enceinte déformable dans le sens de l'accroissement du troisième volume ou dans le sens inverse et exerçant leur action en addition ou en soustraction de l'action des moyens formant ressort pendant la phase systolique et la phase diastolique du cycle cardiaque.

4. Le dispositif des revendications 2 et 3 prises en combinaison, dans lequel les moyens moteurs (38) sont disposés à l'intérieur du corps, et comportent un moteur rotatif (46, 48) coopérant avec une tige (36) solidaire du piston par l'intermédiaire d'une transmission (56, 58, 60) transformant, de manière réversible, le mouvement de rotation du moteur rotatif en un mouvement de translation linéaire de la tige.

5. Le dispositif de la revendication 4, dans lequel la transmission comporte un doigt radial (56, 58) solidaire du rotor du moteur rotatif et coopérant avec une gorge hélicoïdale (60) formée dans un élément cylindrique de l'axe du piston.

6. Le dispositif de la revendication 4, dans lequel le moteur rotatif (46, 48) est un moteur électrique du type moteur-couple.

7. Le dispositif de la revendication 4, dans lequel le corps (32) loge un ensemble étanche avec une paroi frontale rigide formant ledit piston (30) et une paroi latérale en forme de soufflet (66), cet ensemble étanche enfermant lui-même la tige du piston (36), le moteur rotatif (46, 48), la transmission (56, 58, 60) et les moyens formant ressort (40).

**Patentansprüche**

1. Vorrichtung innerhalb der Aorta zur Herzunterstützung, die implantierbar ist, vom Typ Gegendruckballon, wobei der Typ umfasst:

   - eine Pumpe (10), welche einen Körper (12) umfasst, der geeignet ist, in eine Arterie eingeführt zu werden, insbesondere der abwärtslaufenden Aorta, welcher ein erstes Volumen (20) definiert, geeignet um an dieser Stelle durch das Blut passiert zu werden und versehen mit einer elastischen Membran (16), welche zwischen Körper und Membran ein zweites Volumen (18) definiert, getrennt von dem ersten Volumen durch die Membran, wobei das zweite Volumen ein variables Volumen ist, welches das erste Volumen (20) zyklisch und in kontrollierter Weise zu modifizieren erlaubt, wobei letzteres sich verkleinert, wenn das zweite Volumen sich vergrößert, und umgekehrt; und
   - ein Druckgenerator/-Regler (24), welcher eine deformierbare Hülle aufweist, die ein drittes Volumen (34) in fluider Kommunikation mit dem zweiten Volumen (18) definiert,

   wobei die Vorrichtung **dadurch gekennzeichnet ist, dass** der Druckgenerator/-Regler (24) ferne Mittel aufweist, welche eine Feder (40) bilden, die geeignet sind, die deformierbare Hülle gegen eine Ausweitung des dritten Volumens zu belasten, welches aus einer Erhöhung des Drucks in dem zweiten Volumen (18) und korrelativ, in der Hülle, resultiert,
   um der Arterie einen Zuwachs an Elastizität im Verlauf des Herzzyklus zu verschaffen.

2. Vorrichtung gemäß Anspruch 1, in welcher die deformierbare Hülle einen in dem Körper (32) beweglichen Kolben (30) aufweist, wobei die Mittel, die eine Feder (40) bilden, den Kolben in dem Körper in Richtung der Verringerung des dritten Volumens belasten, und der Kolben beweglich zwischen zwei Positionen ist, einer Position (m) des maximalen Volumens und der maximalen Kompression der Feder und einer Position (M) des minimalen Volumens und der minimalen Kompression der Feder.

3. Vorrichtung gemäß Anspruch 1, ferner umfassend Motormittel (38), um in kontrollierter Weise die deformierbare Hülle in der Richtung des Zuwachses des dritten Volumens oder in der umgekehrten Richtung zu belasten und ihre Wirkung zusätzlich auszuüben oder unter Abzug der Wirkung der Mittel, die eine Feder bilden, während der systolischen Phase und der diastolischen Phase des Herzzyklus.

4. Vorrichtung gemäß Ansprüchen 2 und 3 in Kombination, in welcher die Motormittel (38) im Inneren des Körpers angebracht sind und einen rotierenden Motor (46, 48) aufweisen, welcher mit einer Kolbenstange (36) zusammenwirkt, die fest mit dem Kolben über eine Transmission (56, 58, 60) verbunden ist, welche, in umkehrbarer Weise, die Rotationsbewegung des rotierenden Motors in eine Bewegung der linearen Verschiebung der Stange umwandelt.

5. Vorrichtung gemäß Anspruch 4, in welcher die Übertragung einen radialen Finger (56, 58) umfasst, der fest mit dem Rotor des rotierenden Motors verbunden ist und mit einem schraubenförmigen Durchlass (60) zusammenwirkt, welcher in einem zylindrischen Element der Achse des Kolbens ausgebildet ist.

6. Vorrichtung gemäß Anspruch 4, in welcher der rotierende Motor (46, 48) ein elektrischer Motor vom Typ Drehmomenterzeuger ist.

7. Vorrichtung gemäß Anspruch 4, in welcher in dem Körper (32) eine dichte Einheit mit einer starren frontalen Wand, welche den Kolben (30) bildet und einer seitlichen Wand in Form eines Gebläses (66), wobei diese dichte Einheit ihrerseits die Stange des Kolbens (36) einschließt, den rotierenden Motor (46, 48), die Transmission (56, 58, 60) und die Mittel, welche eine Feder (40) bilden.

**Claims**

1. An implantable heart-assist device of the back-pressure balloon type, of the type comprising:

   - a pump (10) including a body (12) adapted to be inserted in an artery, in particular the descending aorta, defining a first volume (20) adapted to have the blood flowing through it and provided with a flexible membrane (16) defining between the body and the membrane a second volume (18) separated from the first volume by the membrane, said second volume being a variable volume making it possible to modify cyclically and in controlled manner the first volume (20), the latter decreasing when the second volume increases, and conversely; and

- a pressure generator/regulator (24) including a deformable enclosure (34) defining a third volume (34) in fluid communication with the second volume (18),

said device being **characterised by** the pressure generator/regulator (24) further including spring-forming means (40) for urging the deformable enclosure against an increase of the third volume resulting from an increase in pressure in the second volume and, correspondingly, in the enclosure, whereby providing additional elastance to the artery during the heart cycle.

2. The device of claim 1, in which the deformable enclosure includes a piston (30) movable in a body (32), the spring-forming means (40) urges the piston in the body in its direction for reducing the third volume, and the piston is movable between two positions, a maximum volume position (m) associated with maximum compression of the spring, and a minimum volume position (M) associated with minimum compression of the spring.

3. The device of claim 1, further comprising motor means (38) for urging the deformable enclosure in controlled manner in the direction for increasing the third volume or in the opposite direction, and exerting its action in addition to or in opposition to the action of the spring-forming means during the systolic phase and the diastolic phase of the heart cycle.

4. The device of claims 2 and 3 taken in combination, in which the motor means (38) are disposed inside the body, and comprise a rotary motor (46, 48) co-operating with a rod (36) secured to the piston via a transmission (56, 58, 60) reversibly transforming the rotary motion of the rotary motor into linear translation motion of the rod.

5. The device of claim 4, in which the transmission has a radial finger (56, 58) secured to the rotor of the rotary motor and co-operating with a helical groove (60) formed in a cylindrical element of the piston rod.

6. The device of claim 4, in which the rotary motor (46, 48) is an electric motor of the torque motor type.

7. The device of claim 4, in which the body (32) houses a sealed assembly with a rigid front wall forming said piston (30) and a side wall in the form of a bellows (66), said sealed assembly itself enclosing the rod of the piston (36), the rotary motor (46, 48), the transmission (56, 58, 60), and the spring-forming means (40).

FIG_1

FIG_2

FIG_3

FIG_4

FIG_5

FIG_6

## FIG_7